# EUROPÄISCHE PATENTSCHRIFT

(11) **EP 1 962 960 B1**
(45) Veröffentlichungstag und Bekanntmachung des Hinweises auf die Patenterteilung: **29.05.2013**
(21) Anmeldenummer: 06830454.2
(22) Anmeldetag: 07.12.2006
(51) Int. Cl.: A61P 11/00, A61K 31/40

(54) **VERFAHREN ZUR MIKRONISIERUNG**
MICRONIZATION METHOD
PROCÉDÉ DE MICRONISATION

(30) Priorität: 14.12.2005 DE 102005059602
(43) Veröffentlichungstag der Anmeldung: 03.09.2008
(60) Teilanmeldung: 10180211.4
(73) Patentinhaber: Boehringer Ingelheim Pharma GmbH & Co.KG, 55218 Ingelheim am Rhein (DE)
(72) Erfinder: RASENACK, Nobert, 79576 Weil am Rhein (DE); WALZ, Michael, 55411 Bingen (DE); GRAEBNER, Hagen, 55218 Ingelheim (DE); TRUNK, Michael, 55218 Ingelheim (DE)
(74) Vertreter: Simon, Elke Anna Maria
(86) Internationale Anmeldenummer: PCT/EP2006/069450
(87) Internationale Veröffentlichungsnummer: WO 2007/068655

(56) Entgegenhaltungen:
- WO-A-02/30928
- WO-A-03/078429
- WO-A-2004/084897
- L. GODET-MORAND ET AL.: POWDER TECHNOLOGY, Bd. 128, 2002, Seiten 306-313, XP002419761 in der Anmeldung erwähnt

## Beschreibung

Die Erfindung betrifft ein Verfahren zur Herstellung einer mikroniserten, wasserfreien Form von (1α,2β,4β,5α,7β)-7-((hydroxydi-2-thienylacetyl)oxy]-9,9-dimethyl-3-oxa-9-azoniatricyclo[3.3.1.0^{2,4}]nonane--bromid.

### Hintergrund der Erfindung

Die Verbindung (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, ist aus der Europäischen Patentanmeldung EP 418 716 A1 bekannt und weist die folgende chemische Struktur auf:

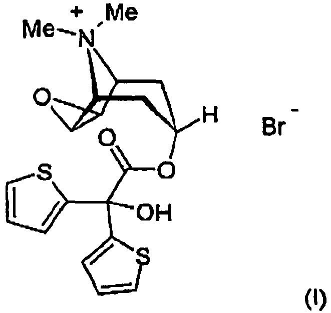

Die Verbindung besitzt wertvolle pharmakologische Elgenschaften und ist unter dem Namen Tiotroplumbromid (BA679) bekannt. Tiotroplumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege. Hierbei können geeignete Inhalationspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalatfonsaerosole erfolgen. Hierzu zählen auch pulverförmige Inhalationsaerosole, die beispielsweise HFA134a, HFA227 oder deren Gemisch als Treibgas enthalten.

Im Hinblick auf die inhalative Applikation von Tiotropiumbromid, ist es erforderlich, den Wirkstoff In feinteiliger (bzw. mikronislerter) Form bereitzustellen. Vorzugsweise weist der Wirkstoff dabei eine mittlere Teilchengröße von 0,5 bis 10µm, bevorzugt von 1 bis 6µm, besonders bevorzugt von 1,5 bis 6µm auf.

Die vorstehend genannten Teilchengrößen werden in der Regel durch Mahlen (sogenanntes Mikronisieren) des Wirkstoffs erzielt. Da als Begleiterscheinung des Mikronisierens trotz der beim Verfahrensablaufs erforderlichen harten Bedingungen ein Abbau des Arzneimittelwirkstoffs weltestgehend vermieden werden muß, stellt eine hohe Stabilität des Wirkstoffs gegenüber dem Mahlvorgang eine unabdingbare Notwendigkeit dar. Dabei muß berücksichtigt werden, daß im Zuge des Mahlvorgangs unter Umständen Veränderungen der Feststoffeigenschaften des Wirkstoffs auftreten können, die einen Einfluß auf die pharmakologischen Eigenschaften der inhalativ zu applizierenden Arzneimittelform haben können.

Verfahren zur Mikronisierung von Arzneimittelwirkstoffen, wie auch dem Tiotropiumbromid, sind als solche im Stand der Technik bekannt. So offenbart beispielsweise die WO 03/078429 ein Verfahren zur Bereitstellung von kristallinem Tiotropiumbromid-Monohydrat Mikronisat.

Aufgabe der vorliegenden Erfindung ist es nun, ein Verfahren bereitzustellen, welches in ökonomischer Form die Bereitstellung von nahezu wasserfreiem mikronisierten Tiotropiumbromid erlaubt, welches zudem den eingangs genannten Anforderungen genügt.

### Detaillierte Beschreibung der Erfindung

Die oben genannten Aufgaben werden durch nachstehend beschriebenes Verfahren gelöst.

Die vorliegende Erfindung betrifft ein Verfahren zur Herstellung von nahezu wasserfreiem Tiotropiumbromid in mikronisierter Form, dadurch gekennzeichnet, dass kristallines Tiotropiumbromid-Monohydrat in einer Gasstrahlmühle zerkleinert wird, in der die Tiotroplumbromid-Monohydrat - Partikel in einem aufgewirbelten Pulverbett zerkleinert werden gemäß Anspruch 1.

Das vorstehend genannte "nahezu wasserfreie Tiotropiumbromid in mikronisierter Form" wird im Rahmen der vorliegenden Erfindung gegebenenfalls auch als nahzu wasserfreies Tiotropiumbromid-Mikronisat, oder auch lediglich als Tiotropiumbromid-Mikronisat bezeichnet.

Im Rahmen der vorliegenden Erfindung ist unter der Bezeichnung "nahezu wasserfrei" kristallines Tiotropiumbromid zu verstehen, welches einen Wassergehalt von ≤ 1,5%, bevorzugtvon ≤ 1,2%, ferner bevorzugt von ≤ 1% aufweist. Besonders bevorzugt beschreibt der Terminus "nahezu wasserfrei" ein kristallines Tiotropiumbromid, welches durch einen Wassergehalt von ≤ 0,8 % gekennzeichnet ist Der Wassergehalt wird im Rahmen der vorliegenden Erfindung mittels der blamperometrischen Titration nach Karl Fischer bestimmt. Detailliertere Angaben dazu sind den experimentellen Beschreibungen der Erfindung zu entnehmen.

Im Rahmen der vorliegenden Erfindung ist unter der Bezeichnung Tiotropiumbromid "in mikronisierter Form" Tiotropiumbromid zu verstehen zu verstehen, welches eine charakteristische Partikelgröße X₅₀ von zwischen 1,0 µm und 3,5 µm, bevorzugt zwischen 1,1 µm und 3,3 µm, besonders bevorzugt zwischen 1,2 µm und 3,0µm und Q_{(5.8)} von größer 60%, bevorzugt größer 70 %, besonders bevorzugt größer 80% aufweist. Dabei bezeichnet der Kennwert X₅₀ den Medianwert der Teilchengröße, unterhalb derer 50% der Teilchenmenge bezüglich der Volumenverteilung der einzelnen Teilchen liegt. Der Kennwert Q_{(5.8)} entspricht der Teilchenmenge der Partikel, die bezogen auf die Volumenverteilung der Partikel unterhalb von 5.8 µm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Im Rahmen der vorliegenden Erfindung ist unter der Bezeichnung kristallines Tiotropiumbromid-Monohydrat diejenige Kristallmodifikation des Tiotropiumbromid-Monohydrats zu verstehen, die durch ein bei der thermischen Analyse mittels DSC auftretendes endothermes Maximum bei 230 ± 5°C (bei einer Heizrate von 10K/min) gekennzeichnet ist Diese Kristallmodifikation ist ferner beschreibbar durch ein IR-Spektrum, das unter anderen bei den Wellenzahlen 3570, 3410, 3105, 1730, 1260, 1035 und 720 cm⁻¹ Banden aufweist und dartiberhinaus gekennzeichnet durch eine einfache monoklinische Zelle mit folgenden Dimensionen: a = 18.0774 Å, b = 11.9711 Å, c = 9.9321 Å, β = 102.691°, V = 2096.96 Å³ (bestimmt durch Einkristallröntgenstrukturanalyse). Verfahren zur Herstellung dieser Modifikation sowie experimentelle Angaben zur Bestimmung der vorstehend genannten Charakteristika sind in der WO 02/30928 offenbart, auf die diesbezüglich Bezug genommen wird. Ein Verfahren zur Herstellung des kristallinen Tiotropiumbromid Monohydrats ausgehend von Tropenol ist ferner der WO 02/051840 zu entnehmen. Nach den vorstehend im Stand der Technik bekannten Verfahren erhältliches kristallines Tiotropiumbromid-Monohydrat wird in das erfindungsgemäße Verfahren eingesetzt. Die Partikelgröße des in das Verfahren eingesetzten kristallinen Tiotropiumbromid-Monohydrats ist für die Durchführbarkeit des erfindungsgemäßen Verfahrens grundsätzlich von untergeordneter Bedeutung. Üblicherweise wird in das erfindungsgemäße Verfahren kristallines Tiotropiumbromid-Monohydrat eingesetzte, welches eine mittlere Teilchengröße im Bereich von etwa 50-1000 µm, bevorzugt von etwa 100-800 µm, besonders bevorzugt von etwas 200-600 µm aufweist.

Die Partikelgrößen werden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Im Rahmen der vorliegenden Erfindung ist unter der Bezeichnung Gasstrahlmühle, eine Mühle zu verstehen, in der es durch hohe Teilchenbeschleunigung aufgrund des expandierenden Mahlgases durch Reibung, Stoss und Schlag zu einer Teilchenzerkleinerung kommt. Neben dieser Mahlfunktion beinhalten Gasstrahlmühlen eine Sichtfunktion. Über diese Sichtfunktion werden kleine von großen Teilchen getrennt, die kleinen Teilchen gelangen in den Produktauffang während die großen Teilchen weiteren Mahlvorgängen unterliegen bis sie ebenfalls fein genug sind um den Sichter zu passieren.

Im Rahmen der vorliegenden Erfindung kommen als Mahlgas Luft, entfeuchtete Luft, fraktionierte Luft, Edelgase, Stickstoff oder Gemische derselben In Frage. Bevorzugt ist fraktionierte Luft, besonders bevorzugt Stickstoff. Unter fraktionierter Luft wird im Rahmen der vorliegenden Erfindung ein Gas verstanden, welches Bestandteile der Luft in aufkonzentrierter aufgereinigter Form enthält. Es eignet sich besonders bevorzugt Stickstoff der Qualitätsklasse Stickstoff 5.0. Diese Qualitätsklasse beschreibt die Reinheit, wobei die Klasse 5.0 eine Reinheit von > 99,999 % (incl. Edelgase) bedeutet mit einem Gehalt an Nebenbestandteilen von ≤ 3 ppm Sauerstoff, ≤ 5 ppm Wasser.

Die im Rahmen der vorliegenden Erfindung zum Einsatz gelangenden Gasstrahlmühlen sind ferner dadurch gekennzeichnet, dass die zu mikronisierenden Partikel in einem aufgewirbelten Pulverbett zerkleinert werden. Dieses sich in der Mahlkammer aufbauende Pulverbett wird In der Literatur auch als Fließbett ("fluidbed") bezeichnet Hier erfolgt wie bei anderen Gasstrahlmühlen auch, eine Teilchenbeschleunigung im Freistrahl, die Zerkleinerung findet jedoch im Einzelstrahl sowie im Gegenstrahl statt. Die im Rahmen der vorliegenden Erfindung zum Einsatz gelangenden Mühlen werden daher auch als Gegenstrahlmühlen oder auch Fließbettgegenstrahlmühlen bezeichnet. In der Mahlkammer baut sich ein aufgewirbeltes Pulverbett auf in dem es durch Schlag, Prall und Reibung zu der Teilchengrößenzerkleinerung kommt. Die Mahldüsen sind hier gegeneinander gerichtet und treffen sich zentral in einem Punkt. Die Sichtfunktion ist separat angelegt mittels eines frei beweglichen separat ansteuerbaren Sichterrades. In Abhängigkeit der Umdrehungsgeschwindigkeit des Sichterrades ergibt sich die Größe der Teilchen, die das Sichterrad passieren können. Das Grobgut wird zurückgewiesen und in der Mahlkammer erneut dem Mahlvorgang zugeführt. Das Feingut passiert das Sichterrad und gelangt in den Produktbehälter.

Die Gegenstrahlmühle ist ein geeignetes Gerät um Substanzen zu zerkleinern. Die Teilchengröße des Produktes kann, wie im Stand der Technik bekannt ist, über die Maschinenparameter gesteuert werden (vgl. Godet-Morand, L. et al., Powder Technology 128 (2002) 306- 313; Heng, P.W.S. et al., S.T.P. Pharma Sciences 10 (1) 445-451 (2000)).

Bei den erfindungsgemäß zum Einsatz gelangenden Gegenstrahlmühlen sind die wesentlichen, variablen Prozessparameter Mahldruck, Düsengröße und Sichterraddrehzahl. Im Rahmen der vorliegenden Erfindung wird der Mahldruck üblicherweise auf einen Wert von 2 - 10 bar, bevorzugt 3 - 8 bar, besonders bevorzugt auf 4 - 6 bar eingestellt. Der Eintrag des Mahlgutes in die Luftstrahlmühle erfolgt mittels eines geeigneten Dosierers (beispielsweise K-Tron (K-Tron GmbH, Geinhausen, Germany). Im Rahmen der vorliegenden Erfindung werden besonders bevorzugt Gegenstrahlmühlen verwendet, die durch 3 gegeneinander gerichtete Mahldüsen gekennzeichnet sind, die jeweils einen Düsendurchmesser von 1,3-2,5 mm, bevorzugt von 1,6 - 2,2 mm, besonders bevorzugt etwa 1,9 mm aufweisen. Unter Düsendurchmesser wird auch im Rahmen der vorliegenden Erfindung der Innendurchmesser der Mahldüse verstanden.

Im Rahmen der vorliegenden Erfindung hat sich eine Sichterraddrehzahl von 5000-22000, bevorzugt 10000 - 20000, besonders bevorzugt 14000-19000, insbesondere von 16000-18000 als geeignet erwiesen. Bei den vorstehend genannten Sichterraddrehzahlen handelt es sich stets um Umdrehungen pro Minute.

Beispielsweise und ohne den Gegenstand der Erfindung darauf zu beschränken, hat sich als eine mögliche Ausführungsform einer erfindungsgemäß einsetzbaren Gegenstrahlmühle das folgende Gerät bewährt Opposed jet-mill AFG 100 (Hosokawa-Alpine, Augsburg, Germany)

Überraschenderweise wurde gefunden, dass mit Hilfe des erfindungsgemäßen Verfahrens direkt eine wasserfreie Modifikation des Tlotropiumbromids in mikronisierter Form erhalten wird. Diese ist unter anderem dadurch gekennzeichnet, dass sie im Röntgenpulverdiagramm unter anderen die charakteristischen Werte d= 5,66 Å; 5,03 Å und 3,99 Å aufweist.

Das gemäß dem erfindungsgemäßen Verfahren zugängliche nahezu wasserfreie Tiotropiumbromid-Mikronisat ist in herausragender Weise zur Darstellung pharmazeutischer Formulierungen geeignet. Besonders bevorzugt kann es zur Herstellung von Inhalationspulvern Verwendung finden.

Derartige Inhalationspulver enthalten wenigstens etwa 0,029 %, vorzugsweise unter 4,81 %, besonders bevorzugt unter 2,89 % des nach vorstehend beschriebenem erfindungsgemäßen Verfahren erhältlichen nahezu wasserfreien Tiotropiumbromid-mikronisats im Gemisch mit einem physiologisch unbedenklichen Hilfsstoff und ggf. weiteren Hilfsstoffen oder auch Wirkstoffen. In Gemischen mit anderen Substanzen kann sich das nach dem beschriebenen erfindungsgemäßen Verfahren erhaltene nahezu wasserfreie Mikronsisat durch besondere Eigenschaften auszeichnen, die die Eigenschaften der Formulierung beeinflussen, so beispielsweise eine verbesserte Stabilität, so beispielsweise eine verbesserte chemische und physikochemische Stabilität.

Als physiologisch unbedenkliche Hilfsstoffe, die zur Darstellung der das nahezu wasserfreie Tiotropiumbromid-Mikronisat enthaltenden Inhalationspulver zur Anwendung gelangen können seien beispielsweise genannt: Monosaccharide (z.B. Glucose oder Arabinose), Disaccharide (z.B, Lactose, Saccharose, Maltose oder Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose, Glucose oder Trehalose, bevorzugt Lactose oder Glucose, insbesondere, aber nicht ausschließlich in Form ihrer Hydrate, bevorzugt ist Als besonders bevorzugt im Sinne der Erfindung gelangt Lactose, höchst bevorzugt Lactosemonohydrat als Hilfsstoff zur Anwendung.

Derartige Inhalationspulver können etwa 0,049 bis etwa 0,96%, bevorzugt etwa 0,096 bis etwa 0,77%, besonders bevorzugt etwa 0,19 bis etwa 0,48% nahezu wasserfreies Tiotropiumbromid-mikronisat enthalten, welches nach vorstehend beschriebenem erfindungsgemäßen Verfahren erhältlich ist und die kennzeichnenden Charakteristika des dadurch erhältlichen Mikronisats aufweist.

Die das nach dem erfindungsgemäßen Verfahren erhältliche, nahezu wasserfreie Tiotropiumbromid-Mikronisat enthaltenden pharmazeutischen Zusammensetzungen bzw. Inhalationspulver können ferner dadurch gekennzeichnet sein, dass der Hilfsstoff eine mittlere Teilchengröße von 10 - 50 µm, einen 10 %-Feinanteil von 0,5 bis 6 µm sowie eine spezifischen Oberfläche von 0,1 bis 2 m²/g aufweist.

Dabei wird unter der mittleren Teilchengröße im hier verwendeten Sinne der 50 % - Wert aus der Volumenverteilung gemessen mit einem Laserdiffraktometer nach der Trockendispersionsmethode verstanden. Analog ist unter dem 10 % - Feinanteil im hier verwendeten Sinne der 10 % - Wert aus der mit einem Laserdiffraktometer gemessenen Volumenverteilung zu verstehen. Mit anderen Worten steht im Sinne der vorliegenden Erfindung der 10%-Feinanteil-Wert für die Teilchengröße, unterhalb derer 10% der Teilchenmenge liegt (bezogen auf Volumenverteilung).

Unter spezifischer Oberfläche wird die massenspezifische Pulveroberfläche verstanden, berechnet aus der N₂-Absorptions-Isotherme, die bei dem Kochpunkt von flüssigem Stickstoff beobachtet wird (Methode von Brunauer, Emmett und Teller).

Bei den im Rahmen.der vorliegenden Erfindung genannten prozentualen Angaben, handelt es sich stets um Gewichtsprozent, soweit nichts Gegenteiliges spezifisch hervorgehoben wird.

In besonders bevorzugten Inhalationspulvern ist der Hilfsstoff durch eine mittlere Teilchengröße von 12 bis 35 µm, besonders bevorzugt von 13 bis 30 µm gekennzeichnet. Ferner sind insbesondere solche Inhalationspulver besonders bevorzugt, in denen der 10 %-Feinanteil etwa 1 bis 4 µm, bevorzugt etwa 1,5 bis 3 µm beträgt.

Alternative pharmazeutische Zusammensetzungen, die das nahezu wasserfreie Tiotropiumbromid enthalten, sind ferner dadurch gekennzeichnet, dass der Hilfsstoff aus einem Gemisch von gröberem Hilfsstoff mit einer mittleren Teilchengröße von 17 bis 50µm, besonders bevorzugt von 20 bis 30µm und feinerem Hilfsstoff mit einer mittleren Teilchengröße von 2 bis 8µm, besonders bevorzugt von 3 bis 7µm besteht. Bevorzugt sind Inhalationspulver bei denen der Anteil von feinerem Hilfsstoff an der Gesamthilfsstoffmenge 3 bis 15%, besonders bevorzugt 5 bis 10% beträgt

Wird im Rahmen der vorliegenden Erfindung auf die Bezeichnung Gemisch Bezug genommen, so ist hierbei stets eine Mischung zu verstehen, die durch Mischen zuvor klar definierter Komponenten erhalten wurde. Entsprechend sind beispielsweise als Hilfsstoffgemisch aus gröberen und feineren Hilfsstoffanteilen nur solche Gemische zu verstehen, die durch Mischen einer gröberen Hilfsstoffkomponente mit einer feineren Hilfsstoffkomponente erhalten werden.

Die gröberen und feineren Hilfsstoffanteile können aus dem chemisch gleichen oder aus chemisch verschiedenen Substanzen bestehen, wobei Inhalationspulver, bei denen der gröbere Hilfsstoffanteil und der feinere Hilfsstoffanteil aus der selben chemischen Verbindung bestehen bevorzugt sind.

Für die Anwendung derartiger Inhalationspulver mittels pulverhaltiger Kapseln werden bevorzugt Kapseln verwendet, deren Hülle aus Gelatine, Cellulosederivaten, Stärke, Stärkederivaten, Chitosan oder synthetischen Kunststoffen gefertigt ist.

Wird Gelatine als Kapselmaterial verwendet, so kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird im Rahmen der vorliegenden Erfindung Gelatine im Gemisch mit PEG, bevorzugt PEG 3350, verwendet. Besonders bevorzugt enthält eine erfindungsgemäße Gelatine-Kapsel PEG in einem Anteil von 1-10% (Gew-%), bevorzugt 3-8 %. Besonders bevorzugte Gelatine-Kapseln enthalten PEG in einem Anteil von 4-6%, wobei ein PEG-Anteil von etwa 5% höchstbevorzugt ist.

Werden Cellulosederivate als Kapselmaterial verwendet, so ist die Verwendung von Hydroxypropylmethylcellulose, Hydroxypropylcellulose, Methylcellulose, Hydroxymethylcellulose und Hydroxyethylcellulose bevorzugt. Besonders bevorzugt wird in diesem Fall Hydroxypropylmethylcellulose (HPMC), besonders bevorzugt HPMC 2910 als Kapselmeterial eingesetzt.

Werden als Kapselmaterial synthetische Kunststoffe eingesetzt, so sind diese bevorzugt ausgewählt aus der Gruppe bestehend aus Polyethylen Polycarbonat, Polyester, Polypropylen und Polyethylenterephthalat. Besonders bevorzugt sind als synthetische Kunststoffmaterialien für die erfindungsgemäßen Inhalationskapseln Polyethylen, Polycarbonat oder Polyethylenterephthalat. Wird Polyethylen als eines der besonders bevorzugten Kapselmaterialien verwendet, gelangt vorzugsweise Polyethylen mit einer Dichte zwischen 900 und 1000 kg/m³, bevorzugt von 940 - 980 kg/m³, besonders bevorzugt von etwa 960 - 970 kg/m³ (high-density Polyethylen) zur Anwendung.

Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet

Ein weiterer Aspekt betrifft vorstehend genannte Kapseln, die vorstehend genanntes Inhalationspulver enthalten. Diese Kapseln können etwa 1 bis 20 mg, bevorzugt etwa 3 bis 15 mg, besonders bevorzugt etwa 4 bis 12 mg Inhalationspulver enthalten. Bevorzugte Formulierungen enthalten 4 bis 6 mg Inhalationspulver. Von gleichrangiger Bedeutung sind Inhaltionskapseln, die die Formulierungen in einer Menge von 8 bis 12 mg enthalten.

Ferner können pharmazeutische Zusammensetzungen, die das nahezu wasserfreie Tiotroplumbromid enthalten, dadurch gekennzeichnet sein, dass sie neben dem nahezu wasserfreien Tiotropiumbromidmikronisat weitere Wirkstoffe enthalten.

Diese Wirkstoffe sind vorzugsweise ausgewählt aus der Gruppe der Betamimetika, Corticosteroide, PDE4-Inhibitoren, LTD4 Antagonisten, EGFR-Hemmer, Dopamin-Agonisten, H1-Antihistaminika oder auch PAF-Antagonisten.

Inhalationspulver, die neben dem nahezu wasserfreien Tiotropiumbromid-Mikronisat einen oder mehrere der vorstehend genannten weiteren Wirkstoffe enthalten können, können in Analogie zu im Stand der Technik bekannten Herstellverfahren erhalten werden. Hierzu sei beispielsweise auf die Offenbarung der Internationalen Patentanmeldungen WO 02/30390, WO 03/017970 oder auch WO 03/017979 verwiesen.

Die das nahezu wasserfreie Mikronisat enthaltenden Inhalationspulver können beispielsweise mittels Inhalatoren appliziert werden, die eine einzelne Dosis aus einem Vorrat mittels einer Messkammer (z.B. gemäß US 4570630A) oder über andere apparative Vorrichtungen (z.B. gemäß DE 36 25 685 A) dosieren. Vorzugsweise werden die Inhalationspulver allerdings in Kapseln abgefüllt (zu sogenannten Inhaletten), die in Inhalatoren wie beispielsweise in der WO 94/28958 beschrieben, zur Anwendung gelangen.

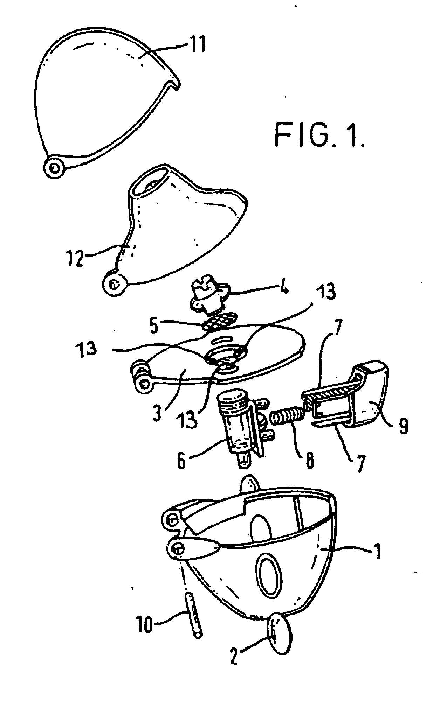

Ferner wird ein Inhalationskit offenbart, bestehend aus einer oder mehrerer der vorstehend beschriebenen, Inhalationspulver enthaltenden Kapseln in Verbindung mit dem Inhalator gemäß Figur 1. Besonders bevorzugt werden die das Inhalationspulver enthaltenden Kapseln mit einem Inhalator appliziert, wie er in Figur 1 dargestellt ist

Dieser Inhalator ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlaßlöcher 13 zur Einstellung des Strömungswiderstandes.

Die das derartige Tiotropiumbromid-mikronisat enthaltenden Inhalationspulver sind durch ein hohes Maß an Homogenität im Sinne der Einzeldosierungsgenaulgkeit gekennzeichnet. Diese liegt in einem Bereich von < 8%, bevorzugt < 6%, besonders bevorzugt < 4%.

Die folgenden, detaillierten experimentellen Ausführungen dienen einer weitergehenden Erläuterung der vorliegenden Erfindung, ohne den Umfang der Erfindung allerdings auf die nachfolgenden beispielhaften Ausführungsformen zu beschränken.

### Experimenteller Teil

### A) Charakterisierungen und Messmethoden

### A.1) Charakterisierung des nahezu wasserfreien Tiotropiumbromid-mikronisats

Das gemäß vorstehender Methodik erhaltene, nahezu wasserfreie Tiotropiumbromid-mikronisat wurde mit Hilfe der Röntgenpulverdiffraktometrie weitergehend untersucht.

Das Röntgenpulverdiffraktogramm wurde im Rahmen der vorliegenden Erfindung aufgenommen mittels eines Stoe & Cie Stadi P X-Ray powder Diffractometers, OED Position Sensitive Detector, CuKα - Strahlung; 40 kV / 40mA; Monochromator: Germanium; 1.5406A; Transmission method; Software package Powdat

In nachstehender Tabelle 1 sind die charakteristischen Peaks und normierten Intensitäten aufgelistet.

**Tabelle 1:**

| **d [Å]** | **Intensität [%]** |
|---|---|
| 10.05 | 46.30 |
| 7.52 | 39.87 |
| 6.79 | 28.41 |
| 6.50 | 17.50 |
| 6.28 | 35.03 |
| 5.96 | 30.59 |
| 5.78 | 13.78 |
| 5.66 | 100.00 |
| 5.37 | 26.01 |
| 5.26 | 45.06 |
| 5.15 | 19.88 |
| 5.03 | 85.00 |
| 4.92 | 15.53 |
| 4.83 | 8.68 |
| 4.73 | 23.69 |
| 4.52 | 34.07 |
| 4.34 | 35.63 |
| 4.26 | 9.39 |
| 4.13 | 16.35 |
| 4.06 | 8.90 |
| 3.99 | 51.41 |
| 3.90 | 11.56 |
| 3.84 | 21.49 |
| 3.76 | 35.19 |
| 3.69 | 41.38 |
| 3.59 | 27.74 |
| 3.54 | 29.64 |
| 3.43 | 30.28 |
| 3.30 | 42.77 |
| 3.15 | 14.26 |
| 3.06 | 21.37 |
| 3.00 | 6.28 |
| 2.95 | 16.87 |
| 2.89 | 22.11 |
| 2.84 | 30.20 |
| 2.72 | 9.82 |
| 2.61 | 9.64 |
| 2.52 | 9.13 |
| 2.50 | 9.13 |
| 2.46 | 8.22 |
| 2.41 | 6.85 |
| 2.37 | 8.49 |
| 2.30 | 7.92 |

### A.2) Bestimmung des Wassergehalts nach Karl-Flscher (Tiotropium Bromid):

| | |
|---|---|
| Titrator | 701 KF-Titrino mit 703 Ti-Stand (Metrohm) mit Doppelplatin-Elektrode; biamperometrische Titration |
| Kalibriersubstanz: | Dinatriumtartratdihydrat (z.B. Riedel de Haen;15.66% Wassergehalt) |
| Titrant: | Karl Fischer Reagenz p.a. (z.B. J. T. Baker; stabilisierte Lösung zur elektrochemischen Titration) |
| Lösungsmittel: | Methanol p.a. (z.B. J. T. Baker) |

### Messmethode:

| | |
|---|---|
| Probenmenge: | ca. 300 mg |
| Rührzeit: | 60 s |

Die Rührzeit vor Beginn der Titration dient dazu, die vollständige Auflösung der Probe zu gewährleisten.

Der Wassergehalt der Probe wird vom Gerät in Prozent berechnet und ausgegeben.

### A.3) Partikelgrößenbestimmung mittels Laserbeugung (Fraunhoferbeugung) Messmethode:

Zur Bestimmung der Partikelgröße wird das Pulver mittels Dispergiereinheit einem Laserbeugungs-Spektrometer zugeführt.

| | |
|---|---|
| Messgerät: | Laser-Beugungs-Spektrometer (HELOS),Fa. Sympatec |
| Software: | WINDOX Version 4.2/ |
| Dispergiereinheit: | RODOS / Dispergierdruck: 3,0 bar |

### Geräteparameter:

| | |
|---|---|
| Detektor: | Multielementdetektor (31 halbkreisförmige Ringe) |
| Methode: | Luft-Dispergierung |
| Brennweite: | 100 mm |
| Messbereich: | RS 0,5/0,9 -175 µm |
| Auswertemodus: | HRLD-Mode |

### Rodos Trockendispergierer

| | |
|---|---|
| Injektor | 4 mm |
| Druck: | 3 bar |
| Injektor-Unterdruck: | maximal (∼100 mbar) |
| Absaugung: | Nilfilsk (Vorlauf 5 s) |
| Dosierer: | Vibri |
| Förderrate: | 40 % (manuelle Steigerung bis 100 %) |
| Betthöhe: | 2 mm |
| Drehzahl: | 0 |

### A.4) Bestimmung der Spezifischen Oberfläche (Mehr-Pkt.-B.E.T.-Methode): Messmethode:

Die Bestimmung der spezifischen Oberfläche erfolgt, indem die Pulverprobe einer Stickstoff/Hellumatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über die Änderung der thermischen Leitfähigkeit des Stickstoff/Heliumgemisches bestimmt und die Oberfläche der Probe über den Flächenbedarf von Stickstoff bestimmt Über diesen Wert und die Probeneinwaage wird die spezifische Oberfläche berechnet.

### Geräte und Materialien:

| | |
|---|---|
| Messgerät: | Multi-Point-BET; Micromeretics TriStar 3000 |
| Ausheizgerät: | VacPrep; Micrometics |
| Mess- und Trockengas: | Stickstoff (5.0) / Helium (4.6) 70/30, Fa. Messer Griesheim |
| Kältemittel: | flüssiger Stickstoff |

### B) Beispiele

### B.1) Darstellung des nahezu wasserfreien Tiotropiumbromid-mikronisats

Zur Mikronisation nach den im folgenden genannten Beispielen wird die Gegenstrahlmühle AFG100 (Hosokawa-Alpine, Augsburg, Germany) verwendet.

Jeweils 500 g Tiotropiumbromidmonohydrat mit einer mittleren Partikelgröße von 450 µm, werden in der Gegenstrahlmühle AFG1 00 unter folgenden Bedingungen mikronisiert:
a) 4,0 bar Mahldruck, 18000 UpM Sichterraddrehzahl, 1,9 mm Düsengröße
b) 6,0 bar Mahldruck, 18000 UpM Sichterraddrehzahl, 1,9 mm Düsengröße
c) 8,0 bar Mahldruck, 16000 UpM Sichterraddrehzahl, 1,3 mm Düsengröße

Das erhaltene Mikronisat ist gekennzeichnet durch eine mittlereTeilchengröße von 1,6 bis 1,8 µm sowie einen Wassergehalt von < 1,0 - 0,5 %

### B.2) Darstellung der Pulverformulierung enthaltend das nahezu wasserfreie Tiotropiumbromid-mikronisat (Referenz)

### Apparatives

Zur Herstellung der Inhalationspulver, die das nahezu wasserfreie Tiotropiumbromid-mikronisat enthalten, können beispielsweise die folgenden Maschinen und Geräte Verwendung finden:

### Mischbehälter bzw. Pulvermischer;

Rhönradmischer 200 L; Typ: DFW80N-4; Hersteller: Firma Engelsmann, D-67059 Ludwigshafen.

### Siebgranulator:

Quadro Comil; Typ: 197-S; Hersteller: Firma Joisten & Kettenbaum, D-51429 Bergisch-Gladbach.

### B.2.1) Pulvermischung A:

Zur Herstellung der Pulvermischung werden 299,41 g Hilfsstoff und 0,59 g nahezu wasserfreies Tiotropiumbromid-mikronisat eingesetzt. In den daraus erhaltenen 300 g Inhalationspulver beträgt der Wirkstoffanteil 0,2 % (bezogen auf Tiotropium).

Über ein Handsieb mit einer Maschenweite von 0,315 mm werden in einen geeigneten Mischbehälter ca. 40-45 g Hilfsstoff vorgelegt. Anschließend werden abwechselnd Tiotropiumbromid-mikronisat in Portionen von ca. 90-110 mg und Hilfsstoff in Portionen von etwa 40-45 g schichtweise eingesiebt. Die Zugabe des Hilfsstoffs und des Wirkstoffs erfolgt in 7 bzw. 6 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß der in Beispiel 1 beschriebenen Vorgehensweise können solche Inhalationspulver erhalten werden, die nach Befüllung der entsprechenden Kunststoffkapseln beispielsweise zu den nachstehenden Inhalationskapseln führen:

### Beispiel 2.1.1:

| | |
|---|---|
| Tiotropiumbromid-mikronisat: | 0,0109 mg |
| Lactose Monohydrat^{*)}: | 5,4891 mg |
| Polyethylen-Kapseln: | 100.0 mg |
| Total: | 105,5 mg |
| mittlere Teilchengröße: | 17,9 µm; |
| 10 %-Feinantell: | 2,3 µm; |
| spezifische Oberfläche: | 0,61 m²/g; |

| | |
|---|---|
| ^{*)} der Hilfsstoff ist durch die folgenden Parameter gekennzeichnet: | |

### Beispiel 2.1.2 :

| | |
|---|---|
| Tiotropiumbromid-mikronisat: | 0,0109 mg |
| Lactose Monohydrat^{*)}: | 5,4891 mg |
| Polyethylen-Kapseln: | 100,0 mg |
| Total: | 105,5 mg |
| mittlere Teilchengröße: | 18,5 µm; |
| 10 %-Feinanteil: | 2,2 µm; |
| spezifische Oberfläche: | 0,83 m²/g; |

| | |
|---|---|
| ^{*)} der Hilfsstoff ist durch die folgenden Parameter gekennzeichnet: | |

### Beispiel 2.1.3:

| | |
|---|---|
| Tiotropiumbromid-mikronisat: | 0,0109 mg |
| Lactose Monohydrat^{*)}; | 5,4891 mg |
| Polyethylen-Kapseln: | 100,0 mg |
| Total: | 105,5 mg |
| mittlere Teilchengröße: | 21,6 µm; |
| 10 %-Feinanteil: | 2,5 µm; |
| spezifische Oberfläche: | 0,59 m²/g; |

| | |
|---|---|
| ^{*)} der Hilfsstoff ist durch die folgenden Parameter gekennzeichnet | |

### Beispiel 2.1.4 :

| | |
|---|---|
| Tiotroplumbromld-mikronisat: | 0,0109 mg |
| Lactose Monohydrat^{*)}; | 5,4891 mg |
| Polyethylen-Kapseln: | 100.0 mg |
| Total: | 105,5 mg |
| mittlere Teilchengröße: | 16,0 µm; |
| 10 %-Feinanteil: | 2,0 µm; |
| spezifische Oberfläche: | 0,79 m²/g; |

| | |
|---|---|
| ^{*)} der Hilfsstoff ist durch die folgenden Parameter gekennzeichnet | |

### Beispiel 2.1.5 :

| | |
|---|---|
| Tiotropiumbromid-mikronisat: | 0,0217 mg |
| Lactose Monohydrat^{*)}: | 5,4783 mg |
| Polyethylen-Kapseln: | 100,0 mg |
| Total: | 105,5 mg |
| mittlere Teilchengröße: | 17,9 µm; |
| 10 %-Feinanteil: | 2,3 µm; |
| spezifische Oberfläche: | 0,61 m²/g; |

| | |
|---|---|
| ^{*)} der Hilfsstoff ist durch die folgenden Parameter gekennzeichnet | |

### Beispiel 2.1.6 :

| | |
|---|---|
| Tiotropiumbromid-mikronisat: | 0, 0217 mg |
| Lactose Monohydrat^{*)}: | 5,4783 mg |
| Polyethylen-Kapseln: | 100,0 mg |
| Total: | 105,5 mg |
| mittlere Teilchengröße: | 18,5 µm; |
| 10 %-Feinanteil: | 2,2 µm; |
| spezifische Oberfläche: | 0,83 m²/g; |

| | |
|---|---|
| ^{*)} der Hilfsstoff ist durch die folgenden Parameter gekennzeichnet: | |

### Beispiel 2.1.7 :

| | |
|---|---|
| Tiotropiumbromid-mikronisat: | 0, 0217 mg |
| Lactose Monohydrat^{*)}: | 5,4783 mg |
| Polyethylen-Kapseln: | 100,0 mg |
| Total: | 105,5 mg |
| mittlere Teilchengröße: | 21,6 µm; |
| 10 %-Feinanteil: | 2,5 µm; |
| spezifische. Oberfläche: | 0,59 m²/g; |

| | |
|---|---|
| ^{*)} der Hilfsstoff ist durch die folgenden Parameter gekennzeichnet: | |

### Beispiel 2.1.8 :

| | |
|---|---|
| Tiotroplumbromid-mikronisat: | 0, 0217 mg |
| Lactose Monohydrat^{*)}: | 5,4783 mg |
| Polyethylen-Kapseln: | 100,0 mg |
| Total: | 105,5 mg |
| mittlere Teilchengröße: | 16,0 µm; |
| 10 %-Feinanteil: | 2,0 µm; |
| spezifische Oberfläche: | 0,79 m²/g; |

| | |
|---|---|
| ^{*)} der Hilfsstoff ist durch die folgenden Parameter gekennzeichnet: | |

### Beispiel 2.1.9 :

| | |
|---|---|
| Tiotroplumbromid-mikronisat: | 0,0054 mg |
| Lactose Monohydrat^{*)}: | 5,4944 mg |
| Polyethylen-Kapseln: | 100,0 mg |
| Total: | 105,5 mg |
| mittlere Teilchengröße: | 17,9 µm; |
| 10 %-Feinanteil: | 2,3 µm; |
| spezifische Oberfläche: | 0,61 m²/g; |

| | |
|---|---|
| ^{*)} der Hilfsstoff ist durch die folgenden Parameter gekennzeichnet: | |

### Beispiel 2.1.10 :

| | |
|---|---|
| Tiotropiumbromid-mikronisat: | 0,0054 mg |
| Lactose Monohydrat^{*)}; | 5,4946 mg |
| Polyethylen-Kapseln: | 100,0 mg |
| Total: | 105,5 mg |
| mittlere Teilchengröße: | 18,5 µm; |
| 10 %-Feinanteil: | 2,2 µm; |
| spezifische Oberfläche: | 0,83 m²/g; |

| | |
|---|---|
| ^{*)} der Hilfsstoff ist durch die folgenden Parameter gekennzeichnet | |

### Beispiel 2.1.11:

| | |
|---|---|
| Tiotropiunibromid-mikronisat: | 0,0054 mg |
| Lactose Monohydrat^{*)}: | 5,4946 mg |
| Polyethylen-Kapseln: | 100,0 mg |
| Total: | 105,5 mg |

| | |
|---|---|
| ^{*)} der Hilfsstoff ist durch die folgenden Parameter gekennzeichnet: | |

| | |
|---|---|
| mittlere Teilchengröße: | 21,6 µm; |
| 10 %-Feinanteil: | 2,5 µm; |
| spezifische Oberfläche: | 0,69 m²/g; |

### Beispiel 2.1.12 :

| | |
|---|---|
| Tiotropiumbromid-mikronisat: | 0,0054 mg |
| Lactose Monohydrat^{*)}: | 5,4946 mg |
| Polyethylen-Kapseln: | 100,0 mg |
| Total: | 105,5 mg |
| mittlere Teilchengröße: | 16,0 µm; |
| 10 %-Feinanteil: | 2,0 µm; |
| spezifische Oberfläche: | 0,79 m²/g; |

| | |
|---|---|
| ^{*)} der Hiffsstoff ist durch die folgenden Parameter gekennzeichnet: | |

### Beispiel 2.1.13:

| | |
|---|---|
| Tiotropiumbromid-mikronisat: | 0,0054 mg |
| Lactose Monohydrat^{*)}: | 9,9946 mg |
| Polyethylen-Kapseln: | 100,0 mg |
| Total: | 110,0 mg |
| mittlere Teilchengröße: | 17,9 µm; |
| 10 %-Feinanteil: | 2,3 µm; |
| spezifische Oberfläche: | 0,61 m²/g; |

| | |
|---|---|
| ^{*)} der Hilfsstoff ist durch die folgenden Parameter gekennzeichnet: | |

### Beispiel 2.1.14 :

| | |
|---|---|
| Tiotropiumbromid-mikronisat: | 0,0109 mg |
| Lactose Monohydrat^{*)}: | 9,9891 mg |
| Polethylen-Kapseln: | 100,0 mg |
| Total: | 110,0 mg |
| mittlere Teilchengröße: | 18,5 µm; |
| 10 %-Feinanteil: | 2,2 µm; |
| spezifische Oberfläche: | 0,83 m²/g; |

| | |
|---|---|
| ^{*)} der Hilfsstoff ist durch die folgenden Parameter gekennzeichnet | |

### Beispiel 2.1.15 :

| | |
|---|---|
| Tiotropiumbromid-mikronisat: | 0, 0217 mg |
| Lactose Monohydrat^{*)}: | 9,9783 mg |
| Polyethylen-Kapseln: | 100,0 mg |
| Total: | 105,5 mg |
| mittlere Teilchengröße: | 18,5 µm; |
| 10 %-Feinanteil: | 2,2 µm; |
| spezifische Oberfläche: | 0,83 m²/g; |

| | |
|---|---|
| ^{*)} der Hilfsstoff ist durch die folgenden Parameter gekennzeichnet | |

### B.2.2) Pulvermischung B:

In den nachfolgenden Beispielen wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden.

In den nachfolgenden Beispielen wird als feinerer Hilfsstoff Lactose-Monohydrat (5µ) verwendet Dieser kann durch gängige Verfahren (Mikronisieren) aus Lactose-Monohydrat 200M erhalten werden. Lactose-Monohydrat 200M kann beispielsweise von der Firma DMV International, 5460 Veghel/NL unter der Produktbezeichnung Pharmatose 200M bezogen werden.

### B.2.2.1.) Herstellung der Hilfsstoffmischung;

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5µm) eingesetzt In den daraus erhaltenen 33,5 kg Hiffsstoffmischung beträgt der Anteil der feineren Hilfsstoffkomponente 5%.

Über einen geeigneten Siebgranulator mit einem Sieb mit einer Maschenweite von 0,5 mm werden in einen geeigneten Mischbehälter ca. 0,8 bis 1,2 kg Lactose Monohydrat für Inhalationszwecke (200M) vorgelegt. Anschließend werden abwechselnd Lactose Monohydrat (5µm) in Portionen von ca. 0,05 bis 0,07 kg und Lactose Monohydrat für Inhalationszwecke (200M) in Portionen von 0,8 bis 1,2 kg schichtweise eingesiebt. Lactose Monohydrat für Inhalationszwecke (200M) und Lactose Monohydrat (5µm) werden in 31 bzw. in 30 Schichten (Toleranz: ±6 Schichten) zugegeben.

Die eingesiebten Bestandteile werden anschließend gemischt (Mischen: 900 Umdrehungen)

### B.2.2.2) Herstellung der Endmischung:

Zur Herstellung der Endmischung werden 32,87 kg der Hilfsstoffmischung (2.1) und etwa 0,125 kg des nahezu wasserfrelen Tiotropiumbromid-mikronisats eingesetzt. In den daraus erhaltenen 33,0 kg Inhalationspulver beträgt der Wirkstoffanteil 0.38%.

Über einen geeigneten Siebgranulator mit einem Sieb mit einer Maschenweite von 0,5 mm werden in einen geeigneten Mischbehälter ca. 1,1 bis 1,7 kg Hilfsstoffmischung (B.2.1) vorgelegt. Anschließend werden abwechselnd Tiotropiumbromid-mikronisat in Portionen von ca. 0,0029 kg und Hilfsstoffmischung (B.2.1) in Portionen von 0,6 bis 0,8 kg schichtweise eingesiebt. Die Zugabe der Hilfsstoffmischung und des Wirkstoffs erfolgt in 46 bzw. 45 Schichten (Toleranz: ± 9 Schichten).

Die eingesiebten Bestandteile werden anschließend gemischt (Mischen: 900 Umdrehungen). Die Endmischung wird noch zweimal über einen Siebgranulator gegeben und anschließend jeweils gemischt (Mischen: 900 Umdrehungen).

Mit der nach B.2.2.2 erhaltenen Mischung oder mit Mischungen, die in analoger Art und Weise erhalten werden, werden Inhalationskapseln der folgenden Zusammensetzungen erhalten:

### Beispiel 2.2.3:

| | |
|---|---|
| Tiotropiumbromid-mikronisat: | 0,0217 mg |
| Lactose Monohydrat (200 M): | 5,2029 mg |
| Lactose Monohydrat (5 µm): | 0,2754 mg |
| Hartgelatinekapsel: | 49,0 mg |
| Total: | 54,5 mg |

### Beispiel 2.2.4:

| | |
|---|---|
| Tiotropiumbromid-mikronisat: | 0,0217 mg |
| Lactose Monohydrat (200 M): | 4,9279 mg |
| Lactose Monohydrat (5 µm): | 0,5504 mg |
| Hartgelatinekapsel: | 49,0 mg |
| Total: | 54,5 mg |

### Beispiel 2.2.5:

| | |
|---|---|
| Tiotropiumbromid-mikronisat: | 0,0217 mg |
| Lactose Monohydrat (200 M): | 5,2029 mg |
| Lactose Monohydrat (5 µm): | 0,2754 mg |
| Polyethylenkapsel: | 100,0 mg |
| Total: | 105,50 mg |

### C) Messtechniken zur Partikelgrößenbestimmung der Hilfsstoffkomponenten, die in B) zur Anwendung gelangen

Nachfolgend wird beschrieben, wie die Bestimmung der mittleren Teilchengröße der verschiedenen Hilfsstoffbestandteile der das nahezu wasserfreie Tiotropiumbromid-mikronisat enthaltenden und nach B) darstellbaren Formulierung erfolgen kann.

### C.1) Partikelgrößenbestimmung von feinteiliger Lactose:

### Messgerät und Einstellungen:

Die Bedienung der Geräte erfolgt in Übereinstimmung mit den Bedienungsanleitungen des Herstellers.

| | |
|---|---|
| Messgerät: | HELOS Laser-Beugungs-Spektrometer, (SympaTec) |
| Dispergiereinheit: | RODOS Trockendispergierer mit Saugtrichter, (SympaTec) |
| Probenmenge: | ab 100 mg |
| Produktzufuhr: | Schwingrinne Vibri, Fa. Sympatec |
| Frequenz d. Vibrationsrinne: | 40 bis 100 % ansteigend |
| Dauer der Probenzufuhr: | 1 bis 15 sek. (im Fall von 100 mg) |
| Brennweite: | 100 mm (Messbereich: 0,9 - 175 µm) |
| Messzeit: | ca. 15 s (im Fall von 100 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

### Probenvorbereitung / Produktzufuhr:

Mind. 100 mg der Prüfsubstanz werden auf einem Kartenblatt eingewogen.

Mit einem weiteren Kartenblatt werden alle größeren Agglomerate zerstoßen. Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

### C.2) Partikelgrößenbestimmung von Laktose 200M :

### Messgerät und Einstellungen

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter, Sympatec |
| Probenmenge: | 500 mg |
| Produktzufuhr: | Vibrationsrinne Typ VIBRI, Sympatec |
| Frequenz d. Vibrationsrinne: | 18 bis 100 % ansteigend |
| Brennweite (1): | 200 mm (Messbereich: 1.8 - 350 µm) |
| Brennweite (2): | 500 mm (Messbereich: 4.5 - 875 µm) |
| Messzeit/Wartezeit: | 10 s |
| Zykluszeit: | 10 ms |
| Start/Stop bei: | 1 % auf Kanal 19 |
| Druck: | 3 bar |
| Unterdruck: | maximal |
| Auswertemodus: | HRLD |

### Probenvorbereitung / Produktzufuhr;

Ca. 500 mg der Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

## Patentansprüche

1. Verfahren zur Herstellung von nahezu wasserfreiem Tiotropiumbromid-Mikronisat, welches einen Wassergehalt von ≤ 1,5% aufweist, **dadurch gekennzeichnet, dass** kristallines Tiotropiumbromid-Monohydrat in einer Gegenstrahlmühle mit separater Sichtfunktion zerkleinert wird, in der die Tiotropiumbromid-Monohydrat - Partikel in einem aufgewirbelten Pulverbett zerkleinert werden und das nahezu wasserfreie Tiotropiumbromid-Mikronisat eine charakteristische Partikelgröße X₅₀ (Medianwert der Teilchengröße) von zwischen 1,0 µm und 3,5 µm und Q_{(5.8)} (Teilchenmenge der Partikel, die bezogen auf die Volumenverteilung der Partikel unterhalb von 5.8 µm liegt) von größer 60%, aufweist, wobei die Teilchenbeschleunigung im Freistrahl, die Zerkleinerung der Partikel dagegen im Einzelstrahl sowie im Gegenstrahl der Gasstrahlmühle erfolgt.

2. Verfahren nach Anspruch 1, **dadurch gekennzeichnet, dass** kristallines Tiotropiumbromid-Monohydrat eingesetzt wird, welches durch ein bei der thermischen Analyse mittels DSC auftretendes endothermes Maximum bei 230 ± 5°C (bei einer Heizrate von 10K/min) gekennzeichnet ist.

3. Verfahren nach Anspruch 1 oder 2, **dadurch gekennzeichnet, dass** das nahezu wasserfreie Tiotropiumbromid-Mikronisat einen Wassergehalt von ≤ 1,2% aufweist.

4. Verfahren nach einem der Ansprüche 1 bis 3, **dadurch gekennzeichnet, dass** als Mahlgas Luft, entfeuchtete Luft, fraktionierte Luft, Edelgase oder StickstoffVerwendung finden.

5. Verfahren nach einem der Ansprüche 1 bis 4, **dadurch gekennzeichnet, dass** der Mahldruck während des Prozesses auf einen Wert von 2 - 10 bar eingestellt ist.

6. Verfahren nach einem der Ansprüche 1 bis 5, **dadurch gekennzeichnet, dass** der Düsendurchmesser der gegeneinander gerichteten Mahldüsen 1,3-2,5 mm beträgt.

7. Verfahren nach einem der Ansprüche 1 bis 6, **dadurch gekennzeichnet, dass** der Mahlprozess bei einer Sichterraddrehzahl von 5000-22000 Umdrehungen pro Minute erfolgt.

## Claims

1. Process for preparing virtually anhydrous micronised tiotropium bromide which has a water content of ≤ 1.5%, **characterised in that** crystalline tiotropium bromide monohydrate is comminuted in a gas jet mill with a separate sifting function, in which the tiotropium bromide monohydrate particles are comminuted in a fluidised powder bed and the virtually anhydrous micronised tiotropium bromide has a characteristic particle size X₅₀ (median value of the particle size) of between 1.0 µm and 3.5 µm and a Q_{(5.8)} (proportion of the particles below 5.8 µm, in relation to the volume distribution of the particles) of more than 60%, the particle acceleration being carried out in free flow, whereas the comminution of the particles takes place in a single current and in countercurrent to the gas jet mill.

2. Method according to claim 1, **characterised in that** crystalline tiotropium bromide monohydrate is used, which is **characterised by** an endothermic peak at 230 ± 5°C (at a heating rate of 10K/min) occurring during thermal analysis using DSC.

3. Method according to claim 1 or 2, **characterised in that** the virtually anhydrous micronised tiotropium bromide has a water content of ≤ 1.2%.

4. Method according to one of claims 1 to 3, **characterised in that** air, dehumidified air, fractionated air, noble gases or nitrogen are used as the grinding gas.

5. Method according to one of claims 1 to 4, **characterised in that** the grinding pressure during the process is adjusted to a value of 2 - 10 bar.

6. Method according to one of claims 1 to 5, **characterised in that** the jet diameter of the grinding jets directed towards one another is 1.3-2.5 mm.

7. Method according to one of claims 1 to 6, **characterised in that** the grinding process is carried out at a screening wheel speed of 5000-22000 revolutions per minute.

## Revendications

1. Procédé de production de micronisat de bromure de tiotropium pratiquement anhydre, qui présente une teneur en eau ≤ 1,5 %, **caractérisé en ce que** le monohydrate cristallin de bromure de tiotropium est broyé dans un broyeur à contre-jets comportant une fonction de séparateur distincte, dans lequel les particules de monohydrate de bromure de tiotropium sont broyées dans un lit de poudre fluidisé et le micronisat de bromure de tiotropium pratiquement anhydre présente une taille de particules caractéristique X₅₀ (valeur médiane de la taille des particules) entre 1,0 µm et 3,5 µm et une Q_{(5,8)} (quantité de particules qui se situent en dessous de 5,8 µm par rapport à la répartition volumique des particules) supérieure à 60 %, l'accélération des particules s'effectuant dans le jet libre, le broyage des particules s'effectuant en revanche dans le jet individuel et dans le contre-jet du broyeur à jet de gaz.

2. Procédé selon la revendication 1, **caractérisé en ce que** l'on utilise du monohydrate de bromure de tiotropium qui est **caractérisé par** un maximum endothermique survenant dans l'analyse thermique par DSC à 230 ± 5°C (pour un taux de chauffage de 10 K/min).

3. Procédé selon la revendication 1 ou 2, **caractérisé en ce que** le micronisat de bromure de tiotropium pratiquement anhydre présente une teneur en eau ≤ 1,2 %.

4. Procédé selon l'une des revendications 1 à 3, **caractérisé en ce que** l'on utilise comme gaz de broyage, de l'air déshumidifié, de l'air fractionné, des gaz inertes ou de l'azote.

5. Procédé selon l'une des revendications 1 à 4, **caractérisé en ce que** la pression de broyage pendant le processus est ajustée à une valeur de 2 à 10 bar.

6. Procédé selon l'une des revendications 1 à 5, **caractérisé en ce que** le diamètre de buse des buses de broyage orientées réciproquement est de 1,3 à 2,5 mm.

7. Procédé selon l'une des revendications 1 à 6, **caractérisé en ce que** le processus de broyage s'effectue avec un nombre de rotations de roue de séparateur de 5000 à 22000 tours par minutes.
